# EUROPEAN PATENT APPLICATION

(11) **EP 0 549 850 A1**
(43) Date of publication of application: **07.07.1993**
(21) Application number: 92108357.2
(22) Date of filing: 18.05.1992
(51) Int. Cl.: A61G 9/00, A61F 5/455

(54) **Urine bag**

(30) Priority: 03.01.1992 DE 9200049 U
(71) Applicant: Yang, Chung-Rong, Tainan Hsein (TW)
(72) Inventor: Yang, Chung-Rong, Tainan Hsein (TW)
(74) Representative: Finck, Dieter, Dr.Ing.

(57) **Abstract**

A urine holder includes a liquid impermeable bag (1), a liquid absorbent material (3) provided in the liquid impermeable bag (1) and a urine guiding member (4) attached to the open top end (11) of the liquid impermeable bag (1).

## Description

This invention relates to a bag, more particularly to a urine bag which is to be used in emergency situations.

Travelers who, for example, ride on a bus or journey to remote places where there are no proper toilet facilities, often encounter the problem of relieving themselves since the human body cannot go for long without urinating. Public urination is an impolite act which disgusts all civilized people.

Therefore, the main object of the present invention is to provide a bag which is adapted to be used for urinating in case of emergency.

Another object of the present invention is to provide a bag which can be easily carried by a traveler and which is adapted to be used where there are no toilet facility.

According to the present invention the bag for urinating in an emergency includes a liquid impermeable bag having a closed bottom end, an open top end and a guiding unit for guiding urine into the impermeable bag and a high absorbent material which is provided in said bag to absorb the incoming urine. The guiding unit is made of plastic material and has the sane shape as that of a urine bowl in a lavatory.

Other features and advantages of the present invention will become apparent in the following detailed description, including drawings, all of which show a non-limiting form of the invention, and of which:
Figure 1 shows an exploded view of a urine bag of the present invention.
Figure 2 shows the urine bag of the present invention in application.

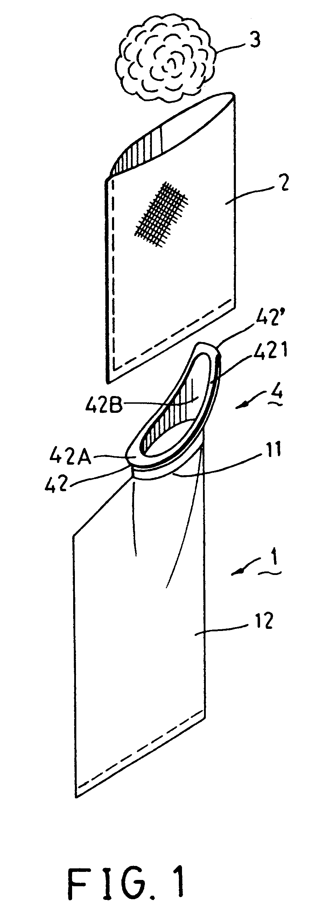

Referring to Figure 1, a urine bag of the present invention includes a liquid impermeable bag (1), a permeable bag (2) for holding an absorbent material (3) and a guiding member (4) for guiding urine into the liquid impermeable bag (1).

As shown in the illustration, the liquid impermeable bag (2) is generally made of plastic material or any other suitable material so long as it can hold the urine therein. It has a closed bottom end, an open top end (11) and an interior (12) for receiving the incoming urine. Some quick-absorbent material (3), such as high absorbent polymer can be put in the permeable bag (2) which is in turn placed inside the liquid impermeable bag (1).

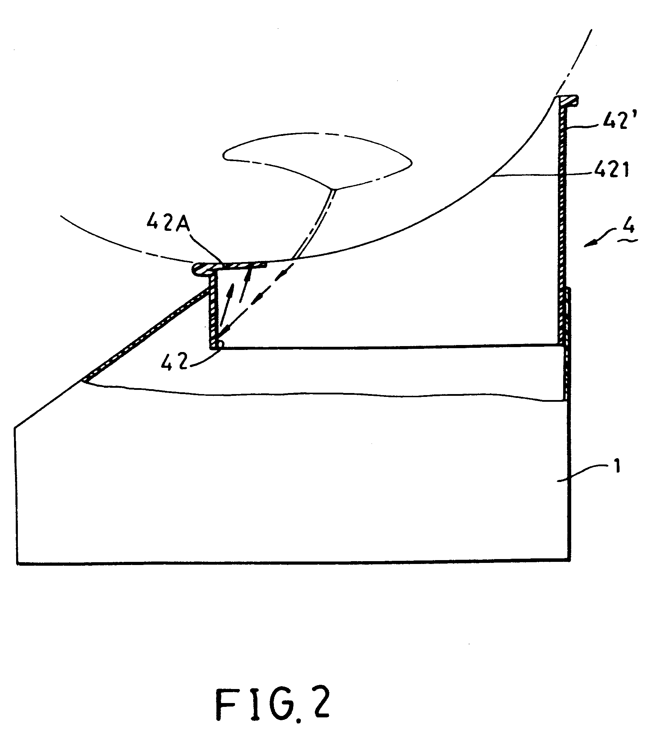

The urine guiding member (4) is made of plastic material and has a lower portion which is attached to the open top end (11) of the liquid impermeable bag (1) by a known heat sealing process so that the urine guiding member is integrally formed with the liquid impermeable bag (1). The guiding member (4) includes a first curved wall section (42), a second curved wall section (42') which is higher than the first curved wall section and two side walls (not figured) interconnecting the two sections as illustrated in Figure 2. An elongated wall (42B) is formed which can prevent the urine from spilling out from the liquid impermeable bag (1). An outwardly extending flange (421) is formed along the periphery of the guiding member (4) for the purpose of facilitating the fitting of the human body to the guiding member while using it. The guiding member (4) further has semi-circle flange (42A) which extends inwardly from the inner most top portion of the first curved section (42) as shown in Figure 2. Its purpose is to prevent the urine from coming out of the liquid impermeable bag (1), especially in the case of use by a female.

## Claims

1. A urine bag characterized by:
a liquid impermeable bag (1) including a closed bottom end and an open top end (11);
a urine guiding member (4) which is made of plastic material and which is fixedly attached to said open top end (11) of said plastic bag (1), said guiding member (4) including a first curved wall section (42), a second curved wall section (42') higher than said first curved wall section (42) and two side walls interconnecting said first curved wall section (42) to said second curved wall section (42') , said first curved wall section having a top portion including a semi-circle flange (42A) which extends towards said second curved wall section (42'); and
a high liquid absorbent polymer (3) provided in said liquid impermeable bag.
